# EUROPEAN PATENT SPECIFICATION

(11) **EP 2 225 366 B1**
(45) Date of publication and mention of the grant of the patent: **22.04.2015**
(21) Application number: 08854121.4
(22) Date of filing: 28.11.2008
(51) Int. Cl.: C12N 7/00, A61K 39/125, A61K 35/76, A61K 39/42, G01N 33/569, C12Q 1/70

(54) **USE OF ENTEROVIRUS FOR DIAGNOSTICS, TREATMENT AND PREVENTION OF CELIAC DISEASE**
VERWENDUNG VON ENTEROVIRUS ZUR DIAGNOSE, BEHANDLUNG UND VORBEUGUNG VON ZÖLIAKIE
UTILISATION D'ENTÉROVIRUS POUR LE DIAGNOSTIC, LE TRAITEMENT ET LA PRÉVENTION DE LA MALADIE COELIAQUE

(30) Priority: 30.11.2007 FI 20075861
(43) Date of publication of application: 08.09.2010
(73) Proprietor: Vactech OY, 33520 Tampere (FI)
(72) Inventor: HYÖTY, Heikki, FI-33230 Tampere (FI); TAURIAINEN, Sisko, FI-33230 Tampere (FI); OIKARINEN, Sami, FI-33500 Tampere (FI); OIKARINEN, Maarit, FI-33500 Tampere (FI); MÄKI, Markku, FI-33180 Tampere (FI); KAUKINEN, Katri, FI-33400 Tampere (FI); HONKANEN, Teemu, FI-40620 Jyväskylä (FI); RANTALA, Immo, FI-13210 Hämeenlinna (FI)
(74) Representative: Kolster Oy Ab
(86) International application number: PCT/FI2008/050698
(87) International publication number: WO 2009/068753

(56) References cited:
- WO-A1-01/00236
- MAWHINNEY, H. ET AL.: 'The immunoglobulin class responses to oral poliovaccine in coeliac disease.' CLINICAL AND EXPERIMENTAL IMMUNOLOGY vol. 21, September 1975, pages 399 - 406
- CARLSSON, A. K. ET AL.: 'Enterovirus infection during pregnancy is not a risk factor for celiac disease in the offspring.' JOURNAL OF PEDIATRIC GASTROENTEROLOGY AND NUTRITION vol. 35, November 2002, pages 649 - 652
- PICHLER, R.: 'Viruses and their roles in immune mediated disease: another piece to this complex puzzle.' JOURNAL OF PEDIATRIC GASTROENTEROLOGY AND NUTRITION vol. 35, November 2002, pages 597 - 599
- VAARALA, O.: 'Candidate molecules that alter the abnormal mucosal immune system in human type 1 diabetes.' IMMUNOLOGY, ENDOCRINE & METABOLIC AGENTS IN MEDICINAL CHEMISTRY vol. 7, no. 3, June 2007, pages 241 - 249
- KAGNOFF, M. F. ET AL.: 'Evidence for the role of a human intestinal adenovirus in the pathogenesis of coeliac disease.' GUT vol. 28, August 1987, pages 995 - 1001
- IVARSSON, A. ET AL.: 'Children born in the summer have increased risk for coeliac disease.' JOURNAL OF EPIDEMIOLOGY AND COMMUNITY HEALTH vol. 57, January 2003, pages 36 - 39
- ARVOLA, T. ET AL.: 'Rectal bleeding in infancy: clinical, allergological, and microbiological examination.' PEDIATRICS vol. 117, no. 4, April 2006, pages E760 - E768
- ROIVAINEN, M.: 'Enteroviruses: new findings on the role of enteroviruses in type 1 diabetes.' THE INTERNATIONAL JOURNAL OF BIOCHEMISTRY & CELL BIOLOGY vol. 38, September 2006, pages 721 - 725
- CHEN, H.-F. ET AL.: 'Oral immunization of mice using transgenic tomato fruit expressing VP1 protein from enterovirus 71.' VACCINE vol. 24, April 2006, pages 2944 - 2951
- C.P. Smith: "Simultaneous Onset of Type 1 Diabetes Mellitus in Identical Infant Twins with Enterovirus Infection", Diabetic Medicine, vol. 15, 1 January 1998 (1998-01-01), pages 515-517, XP055052157, DOI: 10.1002/(SICI)1096-9136(199806)15:6<515::A ID-DIA608>3.0.CO;2-F
- SILANO M ET AL: "Clinical features of chronic C virus hepatitis in patients with celiac disease", EUROPEAN JOURNAL OF CLINICAL MICROBIOLOGY & INFECTIOUS DISEASES, SPRINGER, BERLIN, DE, vol. 28, no. 10, 16 June 2009 (2009-06-16) , pages 1267-1269, XP019743527, ISSN: 1435-4373, DOI: 10.1007/S10096-009-0769-6

## Description

### FIELD OF THE INVENTION

The invention relates to diagnostics, and treatment and prevention of celiac disease. More specifically, the invention relates to a method for diagnosing, and compounds and compositions for treating and preventing celiac disease. The invention further relates to a method for monitoring the treatment of said disease. The invention also relates to the use of substances preventing enteroviruses for the manufacture of a pharmaceutical or a vaccine against this disease.

### BACKGROUND OF THE INVENTION

For some people gluten causes certain diseases, or these diseases are at least in some way associated with gluten intolerance. The best-known of these diseases is celiac disease but there are also other diseases related to gluten-sensitivity that are found on the skin, teeth, bones and in the nervous system and examples of which include, for instance, skin celiac disease, i.e. *dermatitis herpetiformis,* and enamel damage on permanent teeth, osteoporosis and problems with the peripheral and central nervous systems, infertility, pregnancy problems, recurring oral aphthae, articular pain, and arthritis. Celiac disease also involves an increased risk of lymphomas and other malignant tumours.

Celiac disease is caused when the body's immune system identifies certain antigens of gluten in the nutrition and evokes a defence reaction against them. This immunological reaction leads to damages on the intestinal mucous membrane and thus to malabsorption of nutrients. Certain genes, such as genes coding for HLA-DR3 antigen, are involved in contributing to the risk for celiac disease. On the other hand, only a minority of people with a genetic susceptibility actually develop celiac disease, even though they are exposed to gluten of the nutrition. Therefore, there is reason to doubt that besides gluten there is also some other environmental factor that contributes to the starting of an immunological process.

Conventionally, celiac disease is diagnosed by examining intestinal tissue biopsies under the microscope, whereby the intestinal villus in biopsies taken from patients with celiac disease is not as high as normally (Walker-Smith J. A., Guandalini S., Schmitz J., Schmerling D. H., Visakorpi J. K. Revised criteria for diagnosis of coeliac disease. Arch Dis Child 65: 909 - 911, 1990). Nowadays there is also a blood test available, by which celiac-related autoantibodies against tissue transglutaminase (tTGA) are determined (Sulkanen S., Haltunen T., Laurila K., Kolho K. L., Korponay-Szabo I. R., Sarnesto A., Savilahti E., Collin P., Mäki M. Tissue transglutaminase autoantibody enzyme-linked immunosorbent assay in detecting celiac disease. Gastroenterology 115: 1322 - 1328, 1998). A positive blood sample is generally also certified with a conventional biopsy sample.

Mawhinney, H. et al. Clinical and Experimental Immunology 1975, 21:399-406 investigated immunoglobulin class responses to oral polio vaccine in celiac disease and found that both IgA and IgG responses where more frequent in celiac disease patients than controls.

Carlsson, A. K. et al. Journal of Pediatric Gastroenterology and Nutrition 2002, 35:649-652 discloses that viruses may be of importance in the ethiology of celiac disease. They studied whether enterovirus infection during pregnancy was a risk for the disease in the offspring, but found no association.

Pichler, R. Journal of Pediatric Gastroenterology and Nutrition 2002, 35:597-599. D3 relates to the role of viruses in immune mediated diseases. No evidence of a relationship between enteroviruses and celiac disease was reported.

Vaarala, O. Immunology Endocrine & Metabolic Agents in Medicinal Chemistry 2007, 7(3):241-249 suggests that wheat gliadin could be a trigger of intestinal inflammation not associated with celiac disease but present in children with type 1 diabetes.

Kagnoff, M. F. et al. GUT 1987, 28:995-1001 found an increased prevalence of past infection with a human intestinal adenovirus Ad12 among celiac disease patients.

Ivarsson, A. et al. Journal of Epidemiology and Community Health 2003, 57:36-39 found an increased risk of celiac disease in children born in the summer, which was believed to reflect causal environmental exposure with a seasonal pattern. Infections were thought to be of importance, but also non-infectious exposures.

Arvola, T. et al. Pediatrics 2006, 117(4):E760-768 studied the clinical course of rectal bleeding in infants and evaluated the impact of i.a. gut microbiota on the condition. They found an association with enteroviruses in some patients, but the difference was not statistically significant.

WO01/00236 and Roivainen, M. The International Journal of Biochemistry and Cell Biology 2006, 38:721-725 suggest the use of oral polio vaccine against non-polio enterovirus diseases, especially type-1-diabetes.

Chen, H.-F. et al. Vaccine 2006, 24:2944-2951 disclose the use of transgenic tomato fruits expressing VP1 protein from enterovirus 71, which causes hand-foot-and-mouth-disease.[**0005**] The present invention provides a new method for diagnosing celiac disease. The invention also provides means for preventing, treating and monitoring the treatment of celiac disease.

### SUMMARY OF THE INVENTION

The present invention describes for the first time that enterovirus is present on the intestinal mucous membrane of patients with celiac disease and can be discovered from biopsy samples taken from the intestinal mucous membrane. Enteroviruses are common but have never before been suspected to cause celiac disease. In this invention, enterovirus was identified on the mucous membrane of the intestines, and it was further shown that this phenomenon was related to celiac disease. The invention opens up the possibility for using enteroviruses in diagnosing, preventing, treating and monitoring the treatment of celiac disease.

The invention relates to a method for diagnosing celiac disease, characterized by determining the presence of enterovirus in a body sample taken from a subject, whereby the presence of enterovirus in the sample indicates said disease. The presence of enterovirus may be determined directly by demonstrating the complete virus or its component in the sample, or indirectly by determining the immune response caused by the virus.

The invention also relates to an enterovirus or its structural part or an antibody against said virus or structural part for use in vaccinating against celiac disease.

The invention further relates to a method for monitoring the treatment of celiac disease, characterized by determining the presence of enterovirus in in one or more body samples taken from a subject under treatment, whereby the absence or reduction of enterovirus indicates that the treatment has been effective.

The invention further relates to an anti-enterovirus composition for treating or preventing celiac disease, and comprising an anti-enterovirus substance and a pharmaceutically acceptable carrier, wherein the anti-enterovirus substance is selected from the group consisting of pleconaril, pirodavir, rupintrivir, enviroxime, MRL-1237, and MDL-860.

A method for treating or preventing a celiac-related disease is also disclosed, the method comprising administering an effective amount of an anti-enterovirus composition or vaccine to a subject in need of such a treatment.

### DESCRIPTION OF THE FIGURES

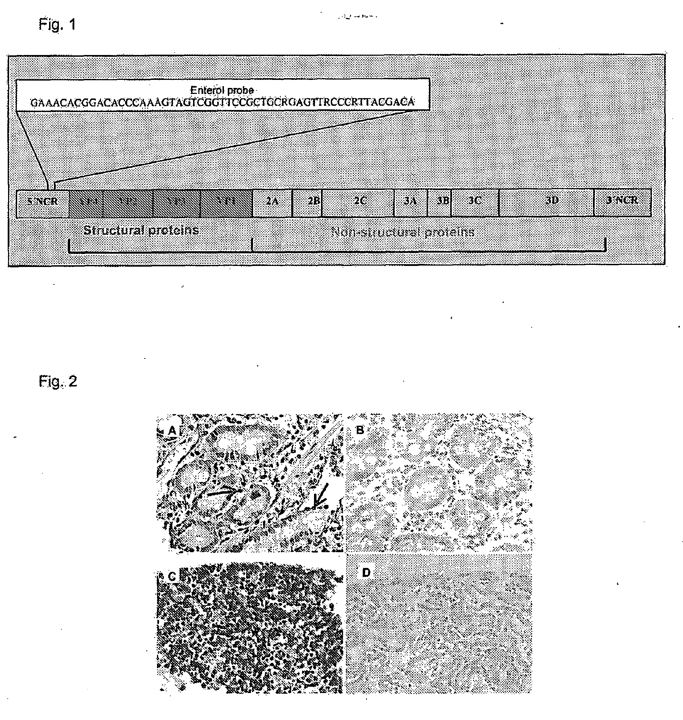

Figure 1 shows the sequence of probes identifying enteroviruses and used in *in situ* hybridization, and the location of the binding region of the probes in the virus genome. The identification was performed by using a combination of eight probes. In the sequence of the probes, letter R refers to either base A or G.
Figure 2 illustrates the detection of the enterovirus genome on the intestinal mucous membrane of a patient with celiac disease by an *in situ* hybridization test. The cells containing enterovirus genome are coloured purple (dark) in the sample (A) of a patient with celiac disease. Some virus-positive cells are denoted by arrows. In Figure (B), the same *in situ* hybridization test is used for analyzing a mucous membrane sample taken from the intestine of a reference patient, whereby no virus-specific staining is detected. The binding of the probe used in the *in situ* hybridization to cultured Green Monkey Kidney (GMK) cells infected with enteroviruses is shown in Figure (C) and to non-infected GMK cells in Figure (D).

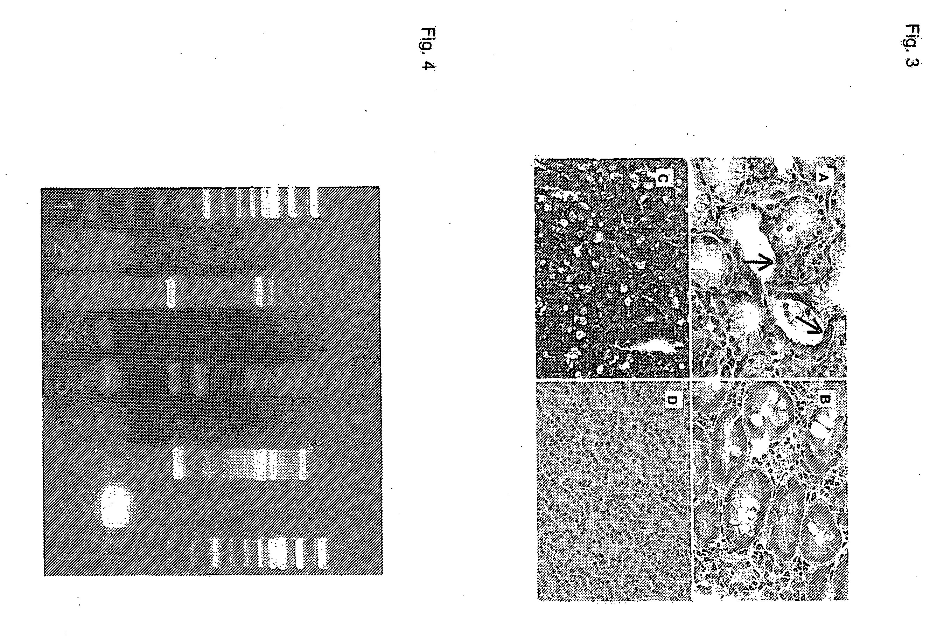

Figure 3 illustrates the detection of the VP1 protein of an enterovirus on the intestinal mucous membrane of a patient with celiac disease by immunohistochemical staining. The protein-containing enterovirus cells are coloured brown (arrows) in the sample (A) of a patient with celiac disease. Figure (B) shows a sample of mucous membrane taken from the intestine of a reference patient and stained with the same antibody, whereby no virus-specific staining is detected. The binding of the antibody to cultured GMK cells infected with enteroviruses is shown in Figure (C) and to non-infected cells in Figure (D).
Figure 4 illustrates the amplification of the enterovirus genome from a biopsy sample taken from the small intestine of a patient with celiac disease by a PCR process. The figure shows the result based on a gel run, wherein the amplification of the enterovirus genome can be concluded from the fact that the product corresponding to the correct molecular weight can be seen in the gel. Samples 1 and 9 are molecular weight standards, and sample 8 is a known enterovirus-positive control sample. The positive sample of a patient with celiac disease is no. 4, where the PCR product is of the same size as in the positive standard. This PCR product also gave a positive signal in a separate solution hybridization test when an enterovirus-specific primer was used (see Table 3). Samples 2 to 3 and 5 to 7 are other research samples.

### DETAILED DESCRIPTION OF THE INVENTION

The invention opens up possibilities for utilizing enteroviruses or components thereof in diagnosing, preventing, treating and monitoring the treatment of celiac disease.

A "celiac-related disease" refers to a disease related to gluten intolerance. The best-known of these is celiac disease, but the disease may also be found on the skin, teeth, bones and in the nervous system, examples of which include, for instance, skin celiac disease, enamel damage on permanent teeth, osteoporosis, problems with the peripheral and central nervous systems, infertility, pregnancy problems, recurring oral aphthae, articular pain, and arthritis. Moreover, celiac disease also involves an increased risk of lymphomas and other malignant tumours. Preferably the celiac-related disease is celiac disease. In this context, the term celiac-related disease also comprises pre-stages of celiac-related diseases e.g. subclinical disease.

Celiac-related diseases including their pre-stages may be diagnosed by detecting enterovirus or its component from a body sample of a subject under examination. A "body sample" may be a biopsy sample, such as a tissue sample, from the patient's intestine in particular and especially the mucous membrane of the small intestine, i.e. *jejunum, duodenum* or *ileum.* Typically the sample is taken from the patient's *duodenum.* The body sample may also be a blood sample or another clinical sample.

A "subject" refers here to a person under examination or treatment.

In this context, an "enterovirus component" refers to any structural part of a virus, such as protein, peptide, other structures, nucleic acid, or other proteins or nucleic acids coded by the virus genome and produced during the virus replication, or any part of the above-mentioned component. The nucleic acid encompassed by the term may be DNA or RNA coding for the entire virus or a negative strand corresponding to the virus RNA, or a fragment of said DNA or RNA molecule.

An "antibody" in this context comprises both complete antibodies and any fragment of an antibody capable of specifically identifying an enterovirus or its component. The antibody may be a polyclonal, monoclonal or recombinant antibody.

A "vaccine" as used herein comprises both vaccines containing viruses, antigens thereof, or nucleic acids coding for them to induce an active immune response in the recipient and vaccines containing an antibody and inducing a passive immune response.

Whole enteroviruses or components thereof may be detected in the sample in various ways by immunology, hybridization techniques or PCR process, for example. Preferably the enterovirus is identified from biopsies taken from the intestinal mucous membrane by using an enterovirus-specific antibody and immunohistochemical detection as well as an enterovirus-specific oligonucleotide probe, which binds to the enterovirus genome of the sample in an *in situ* hybridization test. The virus may also be identified by using an enterovirus-specific RT-PCR process, wherein an enterovirus-specific PCR product is identified by means of a probe binding to the virus genome. On the basis of sequence analysis of the virus genome amplified by PCR, it may be confirmed that the virus belongs to the group of enteroviruses. An enterovirus may also be identified by amplifying its genome by a NASBA (Nucleic Acid Sequence Based Amplification) method or by isolating the virus in a cell culture.

In accordance with the invention, the detection of an enterovirus or its component on the intestinal mucous membrane or other tissues or in blood of a patient can thus be used as a biomarker in the diagnosis of celiac disease, whereby the virus may be identified, for example, by a specific sequence analysis, PCR, an antibody, an oligonucleotide primer or probe, virus isolation or another method specific for that virus, such as NASBA. An enterovirus may also be detected indirectly by measuring antibodies against it or its component in a blood or other clinical sample, whereby the presence of the antibody is a sign of either an ongoing or passed enterovirus infection.

The above-mentioned diagnostic methods can also be used for monitoring the treatment of celiac-related diseases, whereby a body sample may thus be taken, for instance, before and after starting the treatment, and if there are less or no enteroviruses at all after the treatment has been started, it means that the treatment has been effective.

The probes and primers used in the invention are planned suitably in such a manner that they specifically identify a region of the genome of enteroviruses.

The present invention provides an enterovirus or its structural part for use in diagnosing, preventing and monitoring the treatment of celiac disease.

A celiac-related disease can be prevented or treated by antiviral treatment. This treatment may be, for instance, RNA interference based on a siRNA method, a pharmaceutical preventing the growth of enteroviruses, an antibody against the enterovirus or its component, or a molecule preventing the virus from adhering to the cell, such as a soluble cell receptor, or it may be a vaccine against enteroviruses. These treatments may prevent the development of a celiac-related disease or treat a disease that has already developed. A person in need of treatment or prevention is given an "anti-enterovirus composition", which is a composition containing an effective amount of a pharmaceutically active anti-enterovirus substance and pharmaceutically acceptable carrier. The anti-enterovirus substance may be a virus medicament, such as a chemical drug, a cytokine such as interferone-alpha or interferone-beta, siRNA or a peptide that prevents the interaction between the virus and the receptor, or a soluble receptor molecule.

Anti-enterovirus substances are for example, pyridazinamine analogues are known to have antiviral effect against many enteroviruses mediated by blocking the receptor-binding pocket in virus capsid. These drugs can be used to prevent and cure enterovirus infection in gut mucosa. Examples of such drugs include pleconaril and pirodavir and their analogues, which have proved to be efficient against enteroviruses in animal models and in human trials. Other anti-enteroviral drugs which can be used to prevent and treat enterovirus infection in gut mucosa include protease inhibitors (e.g. rupintrivir, enviroxime and their analogies), nucleoside analogues (e.g. ribavirin), and other componds (e.g. MRL-1237, enviroxime and MDL-860) which have shown anti-enteroviral effect in previous publications (De Palma AM, Pürstinger G, Wimmer E, Patick AK, Andries K, Rombaut B, De Clercq E, Neyts J. Potential use of antiviral agents in polio eradication. Emerg Infect Dis. 2008 Apr;14(4):545-51; Collett MS, Neyts J, Modlin JF. A case for developing antiviral drugs against polio. Antiviral Res. 2008 Sep;79(3):179-87).

The vaccines may contain killed or attenuated enterovirus, or virus-like particles, i.e. artificial virus derived from the structural proteins of the virus and lacking the genome, or an enterovirus component capable of inducing a protecting immune response. The vaccine may alternatively contain ready-made antibodies or active fragments thereof for passive immunization. The vaccine may further contain various combinations of the above-mentioned, immunologically active constituents to be administered either simultaneously or at different times. The vaccine may also contain a pharmaceutically acceptable carrier. The enterovirus vaccines described herein can be used for prevention or treatment of celiac-related diseases.

Prevention and treatment of celiac disease is investigated by studying the efficacy of an enterovirus vaccine in the prevention of enterovirus infection in intestinal mucosa in mice. The mice are vaccinated with non-infectious enterovirus particles. Such particles are produced by inactivating the infectivity of enteroviruses using different methods including ultraviolet irradiation, heat-treatment and formalin-treatment or other relevant methodology like using recombinantly produced viral antigens. Mice are vaccinated by these non-infectious enterovirus particles using the oral route in varying doses. In addition, mice are vaccinated using intramuscular injections. Control mice are vaccinated by phosphate-buffered saline using the same protocol. Two to four weeks after the last vaccine dose the mice are challenged with different doses of infective virus using the oral route. The presence of virus in intestinal mucosa is analysed in vaccinated mice and control mice at different time-points after the challenge using RT-PCR, immunohistochemistry and *in situ* hybridization assay. The efficacy of the vaccine is analysed by comparing the presence of virus in intestinal mucosa in vaccine and control groups. A vaccine causing a decrease in enterovirus infection in the intestinal mucosa represents an example of vaccines, which can be used in the prevention and treatment of celiac disease in humans.

Prevention and treatment of celiac disease is also investigated by studying the efficiacy of orally administered immunoglobulins in the prevention of enterovirus infection in intestinal mucosa in mice. The immunoglobulin is produced by immunizing rabbits with highly purified enterovirus, and it includes neutralizing antibodies against the enterovirus serotype which is used to infect the mice. Alternatively, it can be commercially available human immunoglobulin, which includes neutralizing antibodies against several different enterovirus serotypes. Immunoglobulin is given daily using the oral route for 1 week, and the mice are then challenged with infective enterovirus using the oral route and different doses of the virus. Control mice are given phosphate-buffered saline using the same protocol. The presence of virus in intestinal mucosa is analysed in treatment and control groups at different time-points after the challenge using RT-PCR, immunohistochemistry and *in situ* hybridization assay. The efficacy of the treatment is analysed by comparing the presence of virus in intestinal mucosa in treatment and control groups. A decrease in enterovirus infection in the intestinal mucosa in the treatment group represents an example of medication, which can be used in the prevention and treatment of celiac disease in humans.

The present invention is based on a study, in which 42 patients with celiac disease were examined, 22 of which were discovered to have the enterovirus genome on the intestinal mucous membrane. 10 healthy reference patients were analyzed, none of whom had the enterovirus genome on the intestinal biopsies. These results indicate that an enterovirus infection is related to the development of celiac disease and that it may contribute to the body's immunization against gluten in the nutrition. Consequently, preventing an enterovirus infection may prevent the development of celiac disease. Moreover, when an infection of people that have already fallen ill is treated, it is possible to cure the celiac disease that has already developed or to prevent a subclinical celiac disease from developing to a clinical stage.

Enteroviruses useful in the invention can be identified for example by sequencing at least part of the genome of enteroviruses detected in intestinal mucosa of patients with celiac disease. The efficacy of the identified enterovirus can then further be tested by preparing a vaccine or antibodies against them and testing them e.g. in mice, as described above.

Enteroviruses associated with a celiac-related disease include enteroviruses belonging to the genetic cluster II. This genetic cluster of enteroviruses has been described in detail in previous publications (Hyypiä, T., Hovi, T., Knowles, N. J. & Stanway, G. Classification of enteroviruses based on molecular and biological properties. 1997. J Gen Virol 78, 1-11; Pöyry, T., Kinnunen, L., Hyypiä, T., Brown, B., Horsnell, C., Hovi, T. and Stanway, G. Genetic and phylogenetic clustering of enteroviruses. 1996. J Gen Virol 77: 1699-717). The enterovirus infecting the gut mucosa of patients with celiac disease can further be typed using molecular methods such as sequencing the virus genome. These methods can be used e.g. to identify the serotype of the virus e.g. by sequencing the genome regions coding for VP1 protein or other structural proteins of the virus using methods, which have been described in detail previously (Nix, W. A., Oberste, M. S. and Pallansch, M. A. Sensitive, seminested PCR amplification of VP1 sequences for direct identification of all enterovirus serotypes from original clinical specimens. 2006. J Clin Microbiol. 8: 2698-704).

The invention will be illustrated by the following non-limiting examples.

### Example 1. Detection of enterovirus genome on intestinal mucous membrane of patients with celiac disease

A biopsy was taken from the intestinal mucous membrane of patients with celiac disease in connection with gastroscopy. The biopsy was fixed with formaline and cast in paraffin. After this, slices with a thickness of 5 µm were cut from it onto microscope slides. The genome of enteroviruses was detected by *in situ* hybridization using probes that specifically identify a region with a length of 54 bases at the 5'NCR end of the genome of the enteroviruses (SEQ ID NO:1 and Figure 1). The probes are located in the enterovirus genome region (515 to 568 bases in the GenBank sequence with number X80059), which is conserved among different enterovirus serotypes. These probes are planned in such a manner that they adhere to widely known enteroviruses. In addition, the hybridization conditions are optimized so that incompatibility of one or two bases between the enterovirus genome and the probe does not prevent the probe from binding to its target sequence.

With this *in situ* hybridization test, the presence of enterovirus on the mucous membrane of the small intestine was examined among 42 patients with celiac disease and 10 reference patients, who were not discovered to have celiac disease. All reference patients were enterovirus-negative, whereas 52% of people with celiac disease were found to have the enterovirus genome on the intestinal mucous membrane (Figure 2 and Table 1).

**Table 1. Presence of enterovirus genome on intestinal mucous membrane detected with in situ hybridization test**

| | Patients with celiac disease¹ (N=42) | Reference patients² (N=10) |
|---|---|---|
| Enterovirus + | 22³ | 0 |
| Enterovirus - | 20 | 10 |

| | | |
|---|---|---|
| ¹ Patients with celiac disease had celiac disease confirmed by a small intestine biopsy (flat mucous membrane), and tissue transglutaminase antibodies (tTGA) related to celiac disease were detected in the serum. ² Reference patients were patients, from whom a small intestine biopsy was taken because of indefinable abdominal pain but whose biopsies did not reveal celiac disease and who were not discovered to have tissue transglutaminase antibodies (tTGA) related to celiac disease in the serum. ³ Statistical significance P=0.002, when the presence of enterovirus is compared between patients with celiac disease and reference patients (Fisher's exact test). | | |

### Example 2. Detection of structural protein of enterovirus on intestinal mucous membrane of patients with celiac disease

The presence of enterovirus in the intestine of patients with celiac disease was also examined by staining biopsies taken from the intestinal mucous membrane immunohistochemically by using a monoclonal antibody specific for the VP1 protein of enteroviruses. A biopsy was taken from the mucous membrane of the intestines of patients with celiac disease in connection with gastroscopy. The biopsy was fixed with formaline and cast in paraffin. After this, slices with a thickness of 5 µm were cut from it onto microscope slides. The VP1 protein of enteroviruses was identified with a commercial monoclonal antibody (DakoCytomation Denmark A/S, clone 5-D8/1) by using an EnVision⁺ polymer method (DakoCytomation Denmark A/S) and TechMateTM 500 Immunostainer (DakoCytomation Denmark A/S) equipment.

Staining was performed for ten patients with celiac disease and ten reference patients. The VP1 protein of the enterovirus was detected from five (50%) patients with celiac disease and one (10%) reference patient (P=0.07). The results are shown in Figure 3 and Table 2.

**Table 2. Detection of VP1 protein of enterovirus on intestinal mucous membrane by immunohistochemical staining**

| | Patients with celiac disease¹ (N=10) | Reference patients² (N=10) |
|---|---|---|
| Enterovirus + | 5³ | 1 |
| Enterovirus - | 5 | 9 |

| | | |
|---|---|---|
| ¹ Patients with celiac disease had celiac disease certified by a small intestine biopsy (flat mucous membrane), and tissue transglutaminase antibodies (tTGA) related to celiac disease were detected in the serum. ² Reference patients were patients, from whom a small intestine biopsy was taken because of indefinable abdominal pain but whose biopsies did not reveal celiac disease and who were not discovered to have tissue transglutaminase antibodies (tTGA) related to celiac disease in the serum. ³ Statistical significance level P=0.07, when the presence of enterovirus is compared between patients with celiac disease and reference patients (Fisher's exact test). | | |

### Example 3. Detection of enterovirus genome on intestinal mucous membrane of patients with celiac disease by RT-PCR process

Enteroviruses could also be detected with an RT-PCR process by amplifying the genome thereof by means of primers specific for enteroviruses and by detecting the resulting PCR product by means of an enterovirus-specific probe (Table 3). The details of this method are described in the earlier publication (Lönnrot M., Sjöroos M., Salminen K., Maaronen M., Hyypiä T., Hyöty H. Diagnosis of entero- and rhinovirus infections by RT-PCR and time-resolved fluorometry with lanthanide chelate labelled probes. J Med Virol 59: 378 - 384, 1999). An RT-PCR analysis was performed on the basis of biopsies taken from the small intestine of two different patients with celiac disease (in the *in situ* hybridization test these patients were enterovirus-positive). Immediately after the intestine biopsies had been taken, they were frozen to a temperature of -70°C. The enterovirus genome was amplified from one sample of a patient with celiac disease but from none of the samples of reference patients (Figure 4). The base sequence of the genome amplified with the PCR process was determined by a sequence analysis and it belonged to the group of enteroviruses.

**Table 3. Sequences of primers and probe used in an enterovirus RT-PCR process. Locations of the oligonucleotides with relation to enterovirus sequence no. X80059 of GenBank are enclosed in brackets**

| Oligonucleotide | SEQ ID NO: | Base sequence¹ | Location |
|---|---|---|---|
| Forward primer | 2 | 5'-CGGCCCCTGAATGCGGCTAA-3' | (454-473) |
| Reverse primer | 3 | 5'-GAAACACGGACACCCAAAGTA-3' | (548-568) |
| Probe | 4 | 5'-TAITCGGTTCCGCTGC-3' | (534-549) |

| | | | |
|---|---|---|---|
| ¹I refers to inosine | | | |

### Example 4. Target of treatment

The genome of enteroviruses detected in intestinal mucosa of patients with celiac disease was partially sequenced to identify the virus. Sequence analysis was done from the biopsy sample, which was taken from one patient with celiac disease and which was positive for enterovirus genome in screening RT-PCR (see Example 3 and Table 3). For sequencing reaction PCR amplicons of this screening PCR were first purified using the MinElute Gel Extraction kit (Qiagen) and then sequenced with fluorescent dye labelled terminators for both direction using manufacturers protocol (Big-Dye v. 3.1, Applied Biosystems). Sequencing reactions were run on 3730xl DNA Analyzer automatic sequencer (Applied Biosystems) and data was verified with Sequencher (SequencherTM for Windows). Genotype classification of sequenced strain was done using Basic Local Alignment Search Tool in GenBank.

The following enterovirus specific sequence (SEQ ID NO: 5) was obtained from the intestinal biopsy specimen of a patient with celiac disease:

The obtained sequence indicated that the virus belonged to the genetic cluster II of enteroviruses. This genetic cluster of enteroviruses has been described in detail in previous publications (Hyypiä, T., Hovi, T., Knowles, N. J. & Stanway, G. Classification of enteroviruses based on molecular and biological properties. 1997. J Gen Virol 78, 1-11; Pöyry, T., Kinnunen, L., Hyypiä, T., Brown, B., Horsnell, C., Hovi, T. and Stanway, G. Genetic and phylogenetic clustering of enteroviruses. 1996. J Gen Virol 77: 1699-717). The identification of the enterovirus sequence confirms that the virus infecting the gut mucosa of patients with celiac disease is an enterovirus and, more specifically, that it represents genetic cluster II of enteroviruses. Accordingly, the target of preventive and therapeutic treatments and vaccines described herein includes enteroviruses, which belong to this genetic group.

## Claims

1. An enterovirus or a structural part thereof or an antibody against said virus or structural part of said enterovirus for use in vaccinating against celiac disease.

2. The enterovirus or structural part thereof or the antibody against said virus or structural part for use as claimed in claim 1, wherein vaccine comprising different combinations of enterovirus, structural parts thereof or antibodies against said virus or structural part is administered simultaneously or at different times.

3. The enterovirus or structural part thereof or the antibody against said virus or structural part for use as claimed in claim 1 or 2, wherein the structural part is a protein or a nucleic acid.

4. An anti-enterovirus composition for treating or preventing celiac disease, and comprising an anti-enterovirus substance and a pharmaceutically acceptable carrier, wherein the anti-enterovirus substance is selected from the group consisting of pleconaril, pirodavir, rupintrivir, enviroxime, MRL-1237, and MDL-860.

5. A method for diagnosing celiac disease, said method comprising determining the presence of enterovirus in a body sample taken from a subject, whereby the presence of enterovirus in the sample indicates said disease.

6. The method as claimed in claim 5, said method comprising determining a structural part of an enterovirus from the sample.

7. The method as claimed in claim 6, said method comprising determining the structural part being a protein, a peptide or a nucleic acid.

8. The method as claimed in claim 5, said method comprising determining the enterovirus immunohistochemically.

9. The method as claimed in claim 5, said method comprising determining the enterovirus by *in situ* hybridization.

10. The method as claimed in claim 5, said method comprising determining the enterovirus by an RT-PCR process.

11. The method as claimed in claim 5, said method comprising determining the enterovirus by a NASBA method or by isolating the virus in a cell culture.

12. A method for monitoring the treatment of celiac disease, said method comprising determining the presence of enterovirus in one or more body samples taken from a subject under treatment, whereby the absence or reduction of enterovirus indicates that the treatment has been effective.

## Patentansprüche

1. Enterovirus oder ein Strukturteil davon oder ein Antikörper gegen das Virus oder den Strukturteil des Enterovirus zur Verwendung zum Impfen gegen Zöliakie.

2. Enterovirus oder Strukturteil davon oder der Antikörper gegen das Virus oder den Strukturteil zur Verwendung wie in Anspruch 1 beansprucht, wobei ein Impfstoff, der unterschiedliche Kombinationen von Enterovirus, Strukturteilen davon oder Antikörpern gegen das Virus oder den Strukturteil umfasst, gleichzeitig oder zu unterschiedlichen Zeiten verabreicht wird.

3. Enterovirus oder Strukturteil davon oder der Antikörper gegen das Virus oder den Strukturteil zur Verwendung wie in Anspruch 1 oder 2 beansprucht, wobei der Strukturteil ein Protein oder eine Nucleinsäure ist.

4. Anti-Enterovirus-Zusammensetzung zum Behandeln oder Verhüten von Zöliakie, und umfassend eine Anti-Enterovirus-Substanz und einen pharmazeutisch verträglichen Träger, wobei die Anti-Enterovirus-Substanz ausgewählt ist aus der Gruppe bestehend aus Pleconaril, Pirodavir, Rupintrivir, Enviroxim, MRL-1237 und MDL-860.

5. Verfahren zum Diagnostizieren von Zöliakie, wobei das Verfahren das Bestimmen der Anwesenheit eines Enterovirus in einer von einem Probanden genommenen Körperprobe umfasst, wobei die Anwesenheit des Enterovirus in der Probe die Krankheit anzeigt.

6. Verfahren wie in Anspruch 5 beansprucht, wobei das Verfahren das Bestimmen eines Strukturteils von einem Enterovirus aus der Probe umfasst.

7. Verfahren nach Anspruch 6, wobei das Verfahren das Bestimmen des Strukturteils, bei dem es sich um ein Protein, ein Peptid oder eine Nucleinsäure handelt, umfasst.

8. Verfahren wie in Anspruch 5 beansprucht, wobei das Verfahren das immunhistochemische Bestimmen des Enterovirus umfasst.

9. Verfahren wie in Anspruch 5 beansprucht, wobei das Verfahren das Bestimmen des Enterovirus durch in-situ-Hybridisierung umfasst.

10. Verfahren wie in Anspruch 5 beansprucht, wobei das Verfahren das Bestimmen des Enterovirus durch ein RT-PCR-Verfahren umfasst.

11. Verfahren wie in Anspruch 5 beansprucht, wobei das Verfahren das Bestimmen des Enterovirus durch ein NASBA-Verfahren oder durch Isolieren des Virus in einer Zellkultur umfasst.

12. Verfahren zum Monitoring der Behandlung von Zöliakie, wobei das Verfahren das Bestimmen der Anwesenheit eines Enterovirus in einer oder mehreren Körperproben, die von einem Probanden unter Behandlung genommen wurden, umfasst, wobei die Abwesenheit oder Verringerung des Enterovirus anzeigt, dass die Behandlung wirksam war.

## Revendications

1. Entérovirus ou partie structurale de celui-ci ou anticorps dirigé contre ledit virus ou ladite partie structurale dudit entérovirus destiné(e) à être utilisé(e) dans une vaccination contre la maladie coeliaque.

2. Entérovirus ou partie structurale de celui-ci ou anticorps dirigé contre ledit virus ou ladite partie structurale destiné(e) à être utilisé(e) selon la revendication 1, où un vaccin comprenant différentes combinaisons d'entérovirus, de parties structurales de celui-ci ou d'anticorps dirigés contre ledit virus ou ladite partie structurale est administré simultanément ou à des moments différents.

3. Entérovirus ou partie structurale de celui-ci ou anticorps dirigé contre ledit virus ou ladite partie structurale destiné(e) à être utilisé(e) selon la revendication 1 ou 2, où la partie structurale est une protéine ou un acide nucléique.

4. Composition anti-entérovirus destinée au traitement ou à la prévention de la maladie coeliaque, et comprenant une substance anti-entérovirus et un véhicule pharmaceutiquement acceptable, où la substance anti-entérovirus est sélectionnée dans le groupe consistant en le pléconaril, le pirodavir, le rupintrivir, l'enviroxime, le MRL-1237 et le MDL-860.

5. Procédé de diagnostic de la maladie coeliaque, ledit procédé comprenant la détermination de la présence d'un entérovirus dans un échantillon corporel prélevé chez un sujet, moyennant quoi la présence d'un entérovirus dans l'échantillon indique ladite maladie.

6. Procédé selon la revendication 5, ledit procédé comprenant la détermination d'une partie structurale d'un entérovirus à partir de l'échantillon.

7. Procédé selon la revendication 6, ledit procédé comprenant la détermination de la partie structurale qui est une protéine, un peptide ou un acide nucléique.

8. Procédé selon la revendication 5, ledit procédé comprenant la détermination de l'entérovirus par immunohistochimie.

9. Procédé selon la revendication 5, ledit procédé comprenant la détermination de l'entérovirus par une hybridation *in situ.*

10. Procédé selon la revendication 5, ledit procédé comprenant la détermination de l'entérovirus par un processus de RT-PCR.

11. Procédé selon la revendication 5, ledit procédé comprenant la détermination de l'entérovirus par un procédé NASBA ou par un isolement du virus dans une culture cellulaire.

12. Procédé de surveillance du traitement de la maladie coeliaque, ledit procédé comprenant la détermination de la présence d'un entérovirus dans un ou plusieurs échantillons corporels prélevés chez un sujet sous traitement, moyennant quoi l'absence ou la réduction de l'entérovirus indique que le traitement a été efficace.


### SEQUENCE LISTING

<110> vactech oy
<120> Use of enterovirus for diagnostics, treatment and prevention of disease
<130> 2072258PC
<160> 5
<170> PatentIn version 3.3
<210> 1
   <211> 54
   <212> DNA
   <213> Enterovirus sp.
<400> 1
   gaaacacgga cacccaaagt agtcggttcc gctgcrgagt trcccrttac gaca 54
<210> 2
   <211> 20
   <212> DNA
   <213> Enterovirus sp.
<400> 2
   cggcccctga atgcggctaa 20
<210> 3
   <211> 21
   <212> DNA
   <213> Enterovirus sp.
<400> 3
   gaaacacgga cacccaaagt a 21
<210> 4
   <211> 16
   <212> DNA
   <213> Artificial
<220>
   <223> enterovirus probe
<220>
   <221> modified_base
   <222> (3)..(3)
   <223> I
<400> 4
   tagtcggttc cgctgc 16
<210> 5
   <211> 114
   <212> DNA
   <213> Enterovirus sp.
<400> 5
   ggcccctgaa tgcggctaat cctaactgcg gagcacacgt tcgcaagcca gcgagtggtg 60
   tgtcgtaacg ggcaactctg cagcggaacc gactactttg ggtgtccgtg tttc 114